# EUROPÄISCHE PATENTSCHRIFT

(11) **EP 3 359 290 B1**
(45) Veröffentlichungstag und Bekanntmachung des Hinweises auf die Patenterteilung: **01.01.2025**
(21) Anmeldenummer: 16775726.9
(22) Anmeldetag: 04.10.2016
(51) Int. Cl.: B01J 37/03, B01J 21/18, B01J 23/89, B01J 33/00, C07C 29/141, C07C 29/17, C07C 33/02, C07C 33/025

(54) **VERFAHREN ZUR HERSTELLUNG VON RUTHENIUM/EISEN/KOHLENSTOFFTRÄGER-KATALYSATOREN**
METHOD FOR THE PREPARATION OF RUTHENIUM/IRON/CARBON CARRIER CATALYSTS
PROCEDE DE FABRICATION DE CATALYSEURS PORTEURS DE RUTHENIUM/FER/CARBONE

(30) Priorität: 05.10.2015 EP 15188361
(43) Veröffentlichungstag der Anmeldung: 15.08.2018
(73) Patentinhaber: BASF SE, 67056 Ludwigshafen (DE)
(72) Erfinder: SCHAACK, Bernd Bastian, 64625 Bensheim (DE); WANISCH, Helmut, 67454 Haßloch (DE); WUCHER, Barbara, 69514 Laudenbach (DE); HUBER, Sabine, 67240 Bobenheim-Roxheim (DE)
(74) Vertreter: Meissner Bolte Partnerschaft mbB
(86) Internationale Anmeldenummer: PCT/EP2016/073678
(87) Internationale Veröffentlichungsnummer: WO 2017/060243

(56) Entgegenhaltungen:
- EP-A1- 1 317 959
- EP-A2- 0 071 787
- WO-A1-2006/077236
- WO-A1-2011/082967

## Beschreibung

Die vorliegende Erfindung betrifft ein Verfahren zur Herstellung von mit Eisen dotierten Ruthenium/Kohlenstoffträger-Katalysatoren sowie ein Verfahren zur selektiven Flüssigphasenhydrierung von Carbonylverbindungen zu den entsprechenden Alkoholen, insbesondere zur Hydrierung von Citral zu Geraniol bzw. Nerol oder von Citronellal zu Citronellol.

### Stand der Technik:

Aus dem Stand der Technik sind verschiedene Verfahren zur Herstellung von Katalysatoren bekannt. Die Verfahren unterscheiden sich im wesentlichen durch die verwendeten Vorstufen der aktiven Komponenten oder durch die Art der Abscheidung/Behandlung der aktiven Komponenten auf dem KohlenstoffTräger.

EP 0 071 787 offenbart Ruthenium/Eisen/Kohle-Hydrierkatalysatoren sowie deren Herstellung und Verwendung zur selektiven Hydrierung ungesättigter Carbonylverbindungen. Die Herstellung des verwendeten Ru/Fe/Kohlenstoff-Katalysators erfolgt durch Tränkung von Aktivkohlepulver mit Rutheniumchlorid-Lösung, Trocknung und anschliessendem Vermischen mit Eisenoxid. Reduziert wird der Katalysator mit Wasserstoff bei 500°C.

Die Verwendung von Chloriden bereitet jedoch technische Probleme, da Chlorid stark korrosiv wirkt. So muss beim Tränken und Trocknen der aktiven Komponenten in teueren, korrosionsbeständigen Apparaturen gearbeitet werden. Bei der Reduktion entsteht HCl, die den Reduktionsofen schädigen kann und es kann Chlorid auf dem Katalysator verbleiben, das bei Einsatz des Katalysators in der Hydrierung zu Korrosion im Produktionsreaktor führen kann.

Verwendet man statt der Chloride die Nitratsalze des Rutheniums, so kann dies zu einer Sicherheitsproblematik führen, da Nitrat/Kohle-Gemische explosiv sein können. Ein weiterer Nachteil des beschriebenen Verfahrens ist die sequentielle Dotierung mit Fe₂O₃, die einen zusätzlichen Verfahrensschritt erfordert.

Aus dem Stand der Technik sind ebenfalls verschiedene Hydrierverfahren für alpha,beta-ungesättigte Carbonylverbindungen bekannt. Mit den beschriebenen Verfahren und den eingesetzten Katalysatoren ist es jedoch schwierig, hohe Selektivitäten der entsprechenden Alkohole zu erhalten. Bei der Hydrierung von Citral können beispielsweise neben der Aldehydgruppe auch die olefinischen Doppelbindungen hydriert werden oder nur die zur Aldehydgruppe konjugierte Doppelbindung, so dass neben den ungesättigten Alkoholen Geraniol bzw. Nerol auch Nebenprodukte wie Citronellol oder Citronellal entstehen können.

Aus der EP 1 317 959 ist ein Verfahren zur Herstellung von Ruthenium/Eisen/Kohlenstoffträger-Katalysatoren bekannt, bei dem der Katalysator im Wasserstoffstrom bei 400 bis 600°C reduziert wird. Mit diesem Verfahren bzw. den derart hergestellten Katalysatoren werden bereits gute Ergebnisse erzielt.

Sowohl unter ökonomischen als auch ökologischen Gesichtspunkten ist es erwünscht die Katalysatoren und deren Herstellungsverfahren immer weiter zu verbessern, um die Umweltverträglichkeit und Wirtschaftlichkeit weiter zu erhöhen. Insbesondere für grosstechnische Anwendungen ist dies ein erheblicher Aspekt.

### Aufgabe:

Aufgabe der vorliegenden Erfindung war es, ein weiter verbessertes Verfahren zur Herstellung eines Ruthenium/Eisen/Kohlenstoffträger-Katalysators, insbesondere für die Selektivhydrierung von olefinisch ungesättigten Carbonylverbindungen zu den entsprechend ungesättigten Alkoholen, zu entwickeln, ohne die oben beschriebenen Nachteile bzw. mit nochmals verbesserten Ergebnissen.

Der Katalysator sollte eine nochmals verbesserte Katalysatoraktivität und Langzeitstabilität aufweisen sowie auch insbesondere bei der Hydrierung von Citral zu Geraniol/Nerol zu hohen Citralumsätzen und gleichzeitig niedrigen Citronellol-Selektivitäten führen.

Entscheidend soll ferner das mögliche Erreichen eines Vollumsatzes an Citral durch den Katalysator vor einer Selektivität bezüglich der Produkte der Hydrierung zu Geraniol beziehungsweise Nerol sein.

Bei der Herstellung des Katalysators sollte der Einsatz korrosiver Einsatzstoffe wie Chloridsalze bzw. explosiver Zwischenprodukte wie nitratgetränkte Kohlen vermieden werden.

Insbesondere sollten verbesserte Katalysatoren mit ökonomischeren Verfahren hergestellt werden. Auch soll die Verwendung der Katalysatoren bei der Reduktion von Carbonylverbindung ökonomischere Verfahren, insbesondere bezüglich des Energieaufwandes ermöglichen.

Dabei sollten die Katalysatoren insbesondere erhöhte Aktivitäten und verbesserte Langzeitstabilitäten bei Flüssigphasenhydrierungen von Carbonylverbindungen aufweisen.

### Lösung:

Erfindungsgemäß gelöst wurde die Aufgabe durch ein Verfahren zur Herstellung eines Ruthenium/Kohlenstoffträger-Katalysators, enthaltend neben 0,1 bis 10 Gew.-% Ruthenium auf einem Kohlenstoffträger 0,1 bis 5 Gew.-% Eisen, durch
a) Einbringen des Trägers in Wasser
b) Simultane Zugabe der katalytisch aktiven Komponenten in Form von Lösungen ihrer Metallsalze
c) gleichzeitige Auffällung der katalytisch aktiven Komponenten auf den Träger durch Zugabe einer Base
d) Abtrennen des Katalysators von der wässrigen Phase der Trägersuspension
e) Trocknen des Katalysators
f) Reduktion des Katalysators im Wasserstoffstrom bei 100 bis unter 400°C, bevorzugt 120 bis 300°C, besonders bevorzugt 150 bis 250°C, insbesondere 180 bis 220°C
g) Ausbau des Katalysators unter schwer-entflammbaren Flüssigkeiten oder
   passivieren des Katalysators durch Überströmen mit einem verdünnten Sauerstoffstrom
      oder
   passivieren des Katalysators durch Überströmen mit einem verdünnten Sauerstoffstrom und Ausbau des Katalysators unter schwer-entflammbaren Flüssigkeiten.

Verfahren zur selektiven Flüssigphasenhydrierung von Carbonylverbindungen werden ebenfalls beansprucht.

### Begriffsdefinitionen:

Im Rahmen der vorliegenden Erfindung sind alle Mengenangaben, sofern nicht anders angegeben, als Gewichtsangaben zu verstehen.

Im Rahmen der vorliegenden Erfindung bedeutet der Begriff "Zimmertemperatur" eine Temperatur von 20°C. Temperaturangaben sind, soweit nicht anders angegeben, in Grad Celsius (°C).

Sofern nichts anderes angegeben wird, werden die angeführten Reaktionen bzw. Verfahrensschritte bei Normaldruck/Atmosphärendruck, d.h. bei 1013 mbar, durchgeführt.

Im Rahmen der vorliegenden Erfindung schließt die Formulierung "und/oder" sowohl jede beliebige als auch alle Kombinationen der in der jeweiligen Auflistung genannten Elemente ein.

"Oxidation" bedeutet die Erhöhung der Oxidationszahl eines Atoms/Elements, bevorzugt durch Elektronenabgabe.

### Detaillierte Beschreibung:

Gegenstand der vorliegenden Erfindung ist zunächst ein Verfahren zur Herstellung eines Ruthenium/Kohlenstoffträger-Katalysators, enthaltend neben 0,1 bis 10 Gew.-% Ruthenium auf einem Kohlenstoffträger 0,1 bis 5 Gew.-% Eisen, durch
a) Einbringen des Trägers in Wasser
b) Simultane Zugabe der katalytisch aktiven Komponenten in Form von Lösungen ihrer Metallsalze
c) gleichzeitige Auffällung der katalytisch aktiven Komponenten auf den Träger durch Zugabe einer Base
d) Abtrennen des Katalysators von der wässrigen Phase der Trägersuspension
e) Trocknen des Katalysators
f) Reduktion des Katalysators im Wasserstoffstrom bei 100 bis unter 400°C, bevorzugt 120 bis 300°C, besonders bevorzugt 150 bis 250°C, insbesondere 180 bis 220°C.
g) Ausbau des Katalysators unter schwer-entflammbaren Flüssigkeiten oder
   passivieren des Katalysators durch Überströmen mit einem verdünnten Sauerstoffstrom
      oder
   passivieren des Katalysators durch Überströmen mit einem verdünnten Sauerstoffstrom und Ausbau des Katalysators unter schwer-entflammbaren Flüssigkeiten.

In einer Variante der vorliegenden Erfindung wird Schritt f) bei 190 bis 210°C, insbesondere bei 200°C durchgeführt.

Die Schritte (b) und (c) können nach dem erfindungsgemäßen Verfahren sowohl nacheinander als auch simultan durchgeführt werden.

Ein weiterer Gegenstand der Erfindung ist ein Verfahren zur selektiven Flüssigphasenhydrierung von Carbonylverbindungen der allgemeinen Formel I, 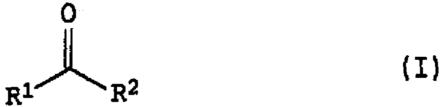 wobei
R¹, R² jeweils unabhängig voneinander gleich oder verschieden sein können und Wasserstoff oder einen gesättigten oder einen ein- oder mehrfach ungesättigten geradkettigen oder verzweigten gegebenenfalls substituierten C₁-C₂₀-Alkylrest, einen gegebenenfalls substituierten Arylrest oder eine gegebenenfalls substituierte heterocyclische Gruppe darstellen
zu den entsprechenden Alkoholen der allgemeinen Formel II 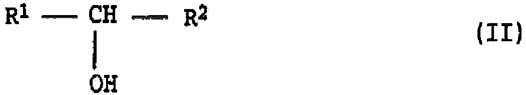
wobei R¹, R² die oben angegebene Bedeutung haben, bei dem man
   (i) einen Katalysator nach einem Verfahren gemäeinem der Ansprüche 1 bis 10 herstellt, und 11
   (ii) die Carbonylverbindung an dem Katalysator hydriert.

Als Carbonylverbindungen können sowohl gesättigte als auch olefinisch ungesättigte Carbonylverbindungen eingesetzt werden.

Unter einem gesättigten oder ein oder mehrfach ungesättigten geradkettigen oder verzweigten C₁-C₂₀-Alkylrest versteht man, wenn nichts anderes angegeben, einen Methyl-, Ethyl-, Propyl-, Isopropyl-, n-Butyl-, i-Butyl-, t-Butyl, Pentyl-, Hexyl-, Heptenyl-, Octyl-, Nonyl-, Decyl-, 1-Propenyl-, 2-Propenyl-, 2-Methyl-2-propenyl-, 1-Pentenyl-, 1-Methyl-2-Pentenyl-, Isopropenyl-, 1-Butenyl-, Hexenyl-, Heptenyl-, Octenyl-, Nonenyl- oder einen Decenylrest bzw. die den weiter unten aufgeführten eingesetzten Verbindungen entsprechenden Reste.

Unter einem Arylrest versteht man einen Benzyl-, Phenyl- oder Naphthylrest.

Unter einer heterocyclischen Gruppe versteht man beispielsweise einen Pyridin-, Pyrimidin-, Pyridazin-, Pyrazin-, Piperazin-, Imidazol-, Furan-, Oxazol-, Isothiazol-, Isoxazol-, 1,2,3-Triazol- oder 1,2,4-Triazol-, Thiazol-, Thiophen- oder Indolring.

Substituenten können Methyl-, Ethyl-, Propyl-, i-Propyl-, Butyl-, t-Butyl-, Fluor-, Chlor-, Brom-, lod-, Nitro- oder Aminoreste bedeuten.

Als gesättigte Carbonylverbindungen werden beispielsweise 3,7-Dimethyloctan-1-al und seine Isomeren, Tetrahydrogeranylaceton, Hexahydrofarnesylaceton, 6-Methylheptanon oder Isovaleraldehyd eingesetzt.

Als olefinisch ungesättigte Carbonylverbindungen können beispielsweise Citronellal, H-Geranylaceton, H-Nerolidol, Methylvinylketon, Mesityloxid, Pseudoionen, Dihydrofarnesylaceton, Lysmeral, Methylhexenon, insbesondere bevorzugt Citronellal oder aber alpha,beta-ungesättigte Carbonylverbindungen, beispielsweise Acrolein, Methacrolein, Crotonaldehyd, Prenal, Farnesal oder Citral, insbesonders bevorzugt Citral eingesetzt werden.

Unter den unter Schritt g) des erfindungsgemäßen Verfahrens genannten schwer-entflammbaren Flüssigkeiten versteht man Flüssigkeiten mit einem Flammpunkt grösser als 80°C, bevorzugt grösser 100°C, beispielsweise Wasser, Geraniol, Pentandiol, Ethylenglykol oder Nerol bzw. Gemische davon, insbesondere bevorzugt Geraniol oder Nerol bzw. Mischungen davon.

In einer Variante der vorliegende Erfindung wird in Schritt g) Wasser als schwerentflammbare Flüssigkeit eingesetzt.

Überraschenderweise führt die simultane Auffällung der Metallsalze der aktiven Komponenten Ruthenium und Eisen zu einer verbesserten Katalysatoraktivität, -selektivität und -standzeit. Durch die Ausfällung der Metalle in Form ihrer Hydroxide werden die im Stand der Technik aufgeführten Probleme der Korrosion bzw. der Explosionsgefahr vermieden.

Als Metallsalze der aktiven Komponenten Ruthenium und Eisen können die Chloride, Nitrate, Nitrosylnitrate, Acetate, Oxide, Hydroxide, Acetylacetonate, bevorzugt die Chloride und Nitrate, eingesetzt werden.

Mit dem erfindungsgemäßen Verfahren kann der Katalysator sowohl als Festbett- als auch als Suspensionskatalysator hergestellt werden.

Unter den Kohlenstoffträger-Materialien versteht man beispielsweise Graphite, Ruße oder Aktivkohle, vorzugsweise jedoch Aktivkohle z.B. NORIT SX Plus^{(R)}. Je nachdem, ob der Katalysator als Suspensions- oder Festbettkatalysator hergestellt werden soll, wird das Kohlenstoffträgermaterial in pulvriger Form oder in Form von Strängen, Kugeln, Splitt etc. eingesetzt. Der Kohlenstoffträger kann vor seiner Dotierung vorbehandelt werden, etwa durch Oxidation mit Salpetersäure, Sauerstoff, Wasserstoffperoxid, Salzsäure, etc.

Die Herstellung des erfindungsgemäßen Katalysators wird im einzelnen wie folgt durchgeführt:
Zur Herstellung eines Suspensionskatalysators wird der Kohlenstoffträger in Wasser suspendiert (Schritt a)) und die so hergestellte Trägersuspension wird entweder ohne weitere Vorbehandlung, d.h. ohne die Einstellung eines bestimmten pH-Wertes, oder durch Einstellen eines pH-Wertes kleiner als 6 mit einer Säure, beispielsweise HNO₃, oder grösser als 8 mit einer Base, beispielsweise NaOH, für das weitere Verfahren verwendet.

In Schritt b) erfolgt die simultane Zugabe der aktiven Komponenten Ruthenium und Eisen in Form von Lösungen ihrer Metallsalze. Die Zugabe erfolgt bevorzugt bei erhöhter Temperatur der Suspension, insbesondere bevorzugt bei einer Temperatur zwischen 50 und 95°C, insbesondere bevorzugt bei einer Temperatur von 70 bis 90°C. Anschliessend wird zur Ausfällung der katalytisch aktiven Komponenten auf den Träger eine Base, beispielsweise Na₂CO₃, NaHCO₃, (NH₄)₂CO₃, NH₃, Harnstoff, NaOH, KOH oder LiOH, bevorzugt NaOH, langsam hinzugefügt und der pH-Wert zwischen 6 und 14, bevorzugt auf 8 bis 12, besonders bevorzugt auf 9, angehoben (Schritt c)). Die Zugabe der Base erfolgt bevorzugt bei erhöhter Temperatur, insbesondere bevorzugt bei einer Temperatur zwischen 50 und 95°C, bevorzugt bei einer Temperatur von 70 bis 90°C. Die Zugabe der Base kann auch gleichzeitig mit der Zugabe der Metallsalzlösung erfolgen, etwa um den pH-Wert der Suspension konstant zu halten, bevorzugt auf einen pH-Wert von 8 bis 14, besonders bevorzugt von 9. Da nach der Fällung Ruthenium und Eisen im wesentlichen als Hydroxide vorliegen, werden Chlorid- bzw. Nitratanionen bei der an die Fällung angeschlossene Waschung und Abtrennung des Katalysators von der wässrigen Phase (Schritt d)) auf einen unproblematisch niedrigen Gehalt ausgewaschen.

Der Filterkuchen wird anschliessend unter Vakuum oder Inertgas getrocknet (e)) und dann wird der Katalysator in einem Wasserstoffstrom, eventuell verdünnt mit einem Inertgas wie Stickstoff, bei 100 bis unter 400°C, bevorzugt 120 bis 300°C, besonders bevorzugt 150 bis 250°C, insbesondere 180 bis 220°C reduziert (f)). Der Wasserstoffgehalt des Wasserstoffstroms beträgt dabei zwischen 5 und 100 Vol-%, bevorzugt zwischen 5 und 50 Vol.-%, in einer Variante kann der Gehalt bei 10 Vol.-% liegen.

Zum Abschluss wird der Katalysator dann nach Abkühlung auf Temperaturen unterhalb von 40°C z.B. unter Wasser oder einer schwer-entflammbaren Flüssigkeit ausgebaut (g)).

Anstelle dieses Ausbaus oder zusätzlich dazu kann eine Passivierung durchgeführt werden.

In einer Variante der vorliegenden Erfindung erfolgt die Passivierung durch Überströmen eines verdünnten Sauerstoffstroms, (g)).

In einer Variante der vorliegenden Erfindung erfolgt die Passivierung bei Zimmertemperatur, indem zuerst reiner Stickstoff übergeleitet und dann langsam, beispielsweise innerhalb einer Stunde, durch Luft ersetzt wird, bis reine Luft übergeleitet wird.

Als verdünnter Sauerstoffstrom kann prinzipiell jeder Gasstrom verwendet werden, der Sauerstoff in nicht überwiegender Menge (d.h. weniger als 50 Vol.-%) enthält. Beispiele sind Inertgas-/Sauerstoffgemische mit einem Sauerstoffanteil von <50 Vol.-%.

Als Inertgase sind insbesondere Stickstoff, Helium, Neon, Argon, CO₂ zu nennen, wobei Stickstoff besonders vorteilhaft ist.

Besonders vorteilhaft sind Gemische, bei denen Anteile von weniger als 10 Vol.-%, bevorzugt weniger als 5 Vol.-% Sauerstoff vorhanden sind, insbesondere etwa Gemische von 1 Vol.-% Sauerstoff in einem Inertgas, wobei das Inertgas in bevorzugten Ausgestaltungen Stickstoff ist.

Es ist ebenfalls möglich Luft als verdünnten Sauerstoffstrom zu verwenden, gegebenenfalls durch zusätzlichen Stickstoff verdünnt.

In einer Variante der vorliegenden Erfindung wird bei 190 bis 210°C, insbesondere 200°C, reduziert (f).

Die Herstellung der Festbettkatalysatoren erfolgt analog dem für den Suspensionskatalysator beschriebenen Verfahren, wobei in Schritt (a) anstelle des pulverförmigen Trägermaterials Stränge, Kugeln, Splitt etc. eingesetzt werden. Die charakteristischen Längen dieser Formkörper (Durchmesser, Länge etc.) liegen in der Regel oberhalb von 1 mm. Bei der Dispergierung der Stränge in Wasser ist darauf zu achten, dass deren mechanische Beanspruchung möglichst gering gehalten wird, um den Abrieb zu minimieren. Vorteilhafterweise werden die Stränge vor Einsatz in der Katalysatorsynthese mit Wasser gewaschen, um schwach anhaftende feinteilige Kohlepartikel zu entfernen.

Die erfindungsgemäßen Katalysatoren enthalten im allgemeinen 0,1 bis 10 Gew.-% Ruthenium auf einem Kohlenstoffträger, vorzugsweise auf Aktivkohle.

Die BET-Oberfläche der Katalysatoren beträgt, entsprechend der zur Herstellung verwendeten Kohlenstoffträger, etwa 100 bis 1500, vorzugsweise etwa 800 bis 1200 m²/g. Die Teilchengrösse der Ruthenium-Kristallite liegt im Wesentlichen unterhalb von 10 nm, bestimmt mittels CO-Adsorption, und entspricht damit den aus der Literatur für Ruthenium/Kohlenstoff-Katalysatoren bekannten Werten.

Die Angaben über die im Katalysator enthaltenen Gew.-% an Ruthenium und Eisen beziehen sich in der vorliegenden Anmeldung immer auf die Trockenmasse des Katalysators.

Die Partikel der Suspensionskatalysatoren hergestellt mit dem erfindungsgemäßen Verfahren haben eine Dichte, die (im Wesentlichen) gleich oder geringer als die Dichte der Mischung ist in welcher die Hydrierung durchgeführt wird, da die Partikel zur Katalyse in Suspension verbleiben und nicht sedimentieren.

Besondere Bedeutung hat der erfindungsgemäß hergestellte Katalysator für die selektive Hydrierung von Carbonylverbindungen, bevorzugt für die selektive Hydrierung von ungesättigten Carbonylverbindungen, insbesondere bevorzugt für die Hydrierung von Citral zu Geraniol bzw. Nerol oder von Citronellal zu Citronellol.

Der erfindungsgemäß hergestellte Katalysator hydriert mit überraschend hoher Selektivität die Aldehyd-Gruppe der Carbonylverbindung.

Das Hydrierverfahren kann sowohl kontinuierlich als auch diskontinuierlich in Suspension oder im Festbett durchgeführt werden. Besonders vorteilhaft ist die kontinuierliche Fahrweise.

Für die Suspensions- oder Festbettvariante bieten sich übliche Reaktorkonzepte an, wie sie z.B. in Ullmann's Encyclopedia of Industrial Chemistry, Sixth Edition, 2000 Electronic Release, beschrieben sind.

Das kontinuierliche bzw. diskontinuierliche Suspensionsverfahren kann beispielsweise wie in EP 0 947 493 bzw. US 5,939,589 beschrieben durchgeführt werden. Der Katalysator wird sowohl in der diskontinuierlichen als auch in der kontinuierlichen Suspensionsfahrweise in feinverteilter Form eingesetzt, wobei die Teilchengrösse kleiner 1 mm, bevorzugt zwischen 1 und 100 µm, besonders bevorzugt zwischen 10 und 50 µm ist, jeweils gemessen mittels Laserbeugung. In einer Variante der vorliegende Erfindung weist die Partikelgrößenverteilung einen d50-Wert zwischen 15 und 25 µm auf. Geeignete Geräte hierfür sind beispielsweise der Mastersizer 2000 oder der Mastersizer 3000, beide von der Firma Malverm.

Für die Festbettvariante wird der Katalysator in für Festbettkatalysatoren üblichen Formen beispielsweise in Strang-, Splitt-, Tabletten- oder Kugelform eingesetzt. Typische Strangdurchmesser liegen zwischen 1 und 5 mm, die Stranglängen liegen bei 1 bis 20 mm. Der Reaktor kann in Riesel- oder Sumpffahrweise betrieben werden.

Die Reaktion wird sowohl bei der Suspensionsfahrweise als auch bei der Festbettvariante drucklos oder unter einem Druck von 1 bis 200 bar, bevorzugt 10 bis 100 bar, insbesondere bevorzugt bei 20 bis 50 bar, durchgeführt. Die Temperaturen liegen zwischen 25 und 200°C, bevorzugt zwischen 60 und 100°C. Die Reaktion kann sowohl mit als auch ohne Lösungsmittel durchgeführt werden. Als Lösungsmittel können niedere Alkohole wie Methanol, Ethanol oder Isopropylalkohol eingesetzt werden. Weiterhin kann bei Bedarf eine organische Base wie Trimethylamin zugesetzt werden.

Die Hydrierung der Carbonylverbindung an den erfindungsgemäß hergestellten Katalysatoren wird vorzugsweise in Gegenwart eines tertiären Amins durchgeführt.

Prinzipiell sind jegliche tertiäre Amine geeignet, so dass es auf deren chemische Natur, sofern sie aufgrund funktioneller Gruppen nicht anderweitig mit den Reaktionspartnern reagieren können, nicht ankommt.

Als Amine kommen beispielsweise die in EP 0 071 787 genannten in Frage.

Die Menge der Amine dabei kann in sehr weiten Bereichen variieren.

In einer Variante der vorliegenden Erfindung beträgt die Menge der Amine 1 bis 5 Gew.-% der Menge der eingesetzten Carbonylverbindung.

Die erfindungsgemäßen/erfindungsgemäß hergestellten Katalysatoren zeigen höhere Langzeitstabilität und Aktivität als solche gemäß EP 1 317 959.

Es werden mit den erfindungsgemäßen/erfindungsgemäß hergestellten Katalysatoren schneller höhere Umsätze erreicht als mit den Katalysatoren gemäß EP 1 317 959 und die Umsätze liegen bei fortgesetzter Benutzung über denjenigen der EP 1 317 959 (vgl. Figur 3).

Diese Ergebnisse waren überraschend, da bei der Reduktionstemperatur von bei 100 bis unter 400°C, bevorzugt 120 bis 300°C, besonders bevorzugt 150 bis 250°C, insbesondere 180 bis 220°C eine Reduktion der Eisenverbindung erfolgt. Denn obwohl Eisen bei diesen Temperaturen, insbesondere bei 180 bis 220°C nicht reduziert werden kann, führt die Kombination mit Ruthenium dennoch schon bei diesen Temperaturen zu einer Reduktion - dies war bisher nicht bekannt und folgerichtig wurden im Stand der Technik höhere Temperaturen angesetzt. Der reduziert vorliegende Katalysator konnte durch Röntgenphotoelektronenspektroskopie (XPS) sowie Temperatur-programmierte Reduktion (TPR) nachgewiesen werden.

Durch die erhöhte Aktivität und Langzeitstabilität der erfindungsgemäßen/erfindungsgemäß hergestellten Katalysatoren können die Kapazitäten und Laufzeiten der Produktionsanlagen erhöht werden, was, insbesondere bei großtechnischen Anlagen, immense ökologische und ökonomische Vorteile darstellt.

Ein weiterer Vorteil der vorliegenden Erfindung ist dass durch die geringere Reduktionstemperatur der reduzierte Katalysator auch ohne Passivierung direkt in Wasser eingefüllt werden kann, was Zeit und Kosten spart, also ein erheblicher verfahrenstechnischer und ökologisch/ökonomischer Vorteil ist. Weiterhin wird für die gleiche Menge Produkt weniger Katalysator benötigt.

Die verschiedenen Ausgestaltungen der vorliegenden Erfindung, z.B. aber nicht ausschließlich diejenigen der verschiedenen abhängigen Ansprüche, können dabei in beliebiger Art und Weise miteinander kombiniert werden.

### Figurenbeschreibung:

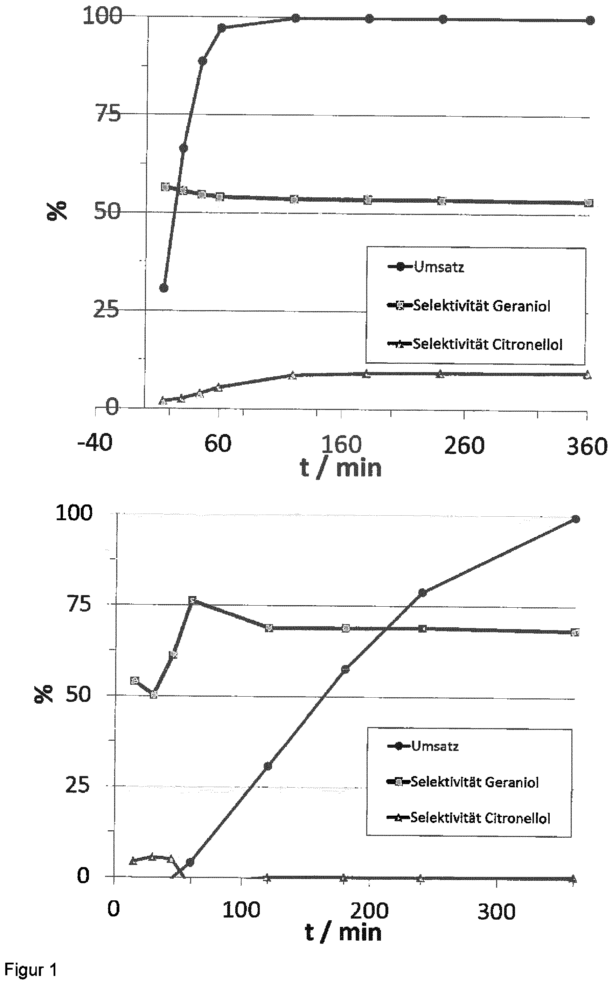

Figur 1:
   Diese Figur zeigt Katalysator-Rezyklierung mit einem erfindungsgemäßen Katalysator, reduziert bei 200°C (hergestellt gemäß Beispiel 3).
   Dieser Katalysator ist aktiv genug, um sogar beim vierten Zyklus nach 360 Minuten einen vollständigen Umsatz zu erreichen (Figur 1, unten). Im ersten Zyklus wurde die vollständige Umsetzung bereits nach 100 Minuten erreicht (Figur 1, oben).
Figur 2:
   Diese Figur zeigt Katalysator-Rezyklierung mit einem Katalysator gemäß EP 1 317 959, reduziert bei 500°C (hergestellt gemäß Vergleichsbeispiel 2).

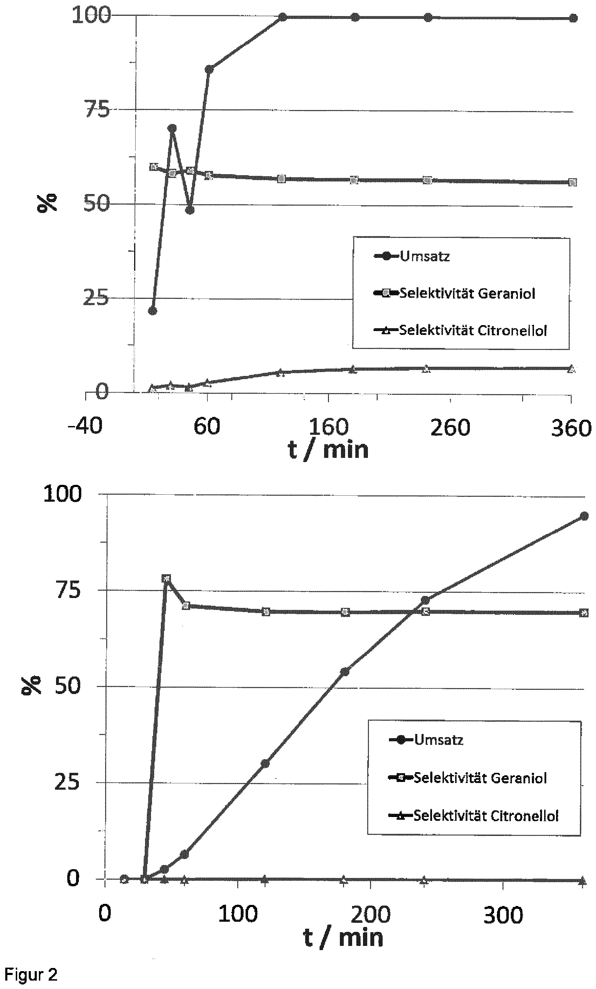

Mit dem bei 500°C reduzierten Katalysator wird beim vierten Zyklus nach 360 Minuten *kein* vollständiger Umsatz erreicht (Figur 2, unten). Im ersten Zyklus wurde die vollständige Umsetzung erst nach 120 Minuten erreicht (Figur 2, oben).

Die Erfindung wird nun unter Bezugnahme auf die folgenden nicht-limitierenden Beispiele erläutert.

### Beispiele:

### Beispiel 1 (Analog Beispiel 1A bis 1C der EP 1 317 959)

A) 100 g Aktivkohle wurden mit 500 ml konzentrierter HNO₃ versetzt und im 1-Liter-Kolben bei 80°C 6 Stunden lang gerührt. Nach Abkühlung wurde filtriert und der Filterkuchen mit 10 Liter destilliertem Wasser gewaschen.
   Die feuchte Kohle wurde wieder in den Rührkessel gegeben, mit 2,5 Liter Wasser suspendiert und unter Rückflusskühlung auf 80°C erhitzt. Dann wurde innerhalb von 120 Minuten unter Rühren eine Lösung aus 13,11 g Rutheniumchlorid und 5,15 g Eisenchlorid in 375 ml Wasser zugetropft. Der pH-Wert der Suspension betrug nach Zugabe der Metallsalzlösung 1,4. Durch Zugabe von 1-M-Natronlauge wurde dann der pH-Wert durch langsames Zutropfen auf 9 angehoben; hierzu wurden etwa 400 ml NaOH verbraucht. Anschliessend wurde 1 Stunde nachgerührt und dann abgekühlt. Der Katalysator wurde auf eine Glasfilternutsche überführt, mit insgesamt 40 Liter Wasser gewaschen und im Vakuumtrockenschrank für 6 Stunden bei 80°C getrocknet. Das getrocknete Pulver wurde dann in einem Drehkugelofen in einem Strom aus 70% H₂ und 30% N₂ bei 200°C für 3 Stunden reduziert. Nach Ende der Reduktion wurde unter Stickstoff abgekühlt und mit einer Gasmischung aus 1% Sauerstoff in Stickstoff passiviert. Der fertige Katalysator hatte einen Chloridgehalt von unter 0,05 Gew.-%. Weiterhin werden folgende Gehalte (Gew.-%) bestimmt: Na: 2,8, Ru: 5,2, Fe: 1,1.
B) Es wurde gearbeitet wie in A beschrieben, jedoch wurde anstelle von Ruthenium- und Eisenchlorid Rutheniumnitrosylnitrat und Eisen-(III)-Nitrat verwendet. Der fertige Katalysator hatte einen Ruthenium-Gehalt von 5,1 Gew.-%, einen Eisen-Gehalt von 1,1 Gew.-%, einen Nitrat-Gehalt <0,01 Gew.-% und einen Na-Gehalt von 2,1 Gew.-%.
C) Es wurde gearbeitet wie in A beschrieben, jedoch wurden geringere Ruthenium- und Eisengehalte auf die Aktivkohle aufgebracht. Der fertige Katalysator hatte einen Ruthenium-Gehalt von 2,8 Gew.-%, einen Eisen-Gehalt von 0,54 Gew.-%, einen Chlorid-Gehalt von 0,02 Gew.-% und einen Na-Gehalt von 3,8 Gew.-%.
D) 110 g der Aktivkohle Norit SX Plus^{(R)} wurden ohne weitere Vorbehandlung in einen Rührkolben mit 2 Liter Wasser gegeben, suspendiert und unter Rückfluss auf 80°C erhitzt. Dann wurde der pH-Wert durch Zugabe von wässriger NaOH (1 mol/l) auf 9 angehoben. Innerhalb einer Stunde wurde dann bei 80°C 300 ml einer Lösung von Ruthenium-Nitrosylnitrat und Eisennitrat (Konzentration entsprechend 5,85 g Ru und 1,17 g Fe) zugetropft, gleichzeitig wurde durch simultane Zugabe von wässriger NaOH der pH-Wert auf ca. 9 gehalten. Es wurde eine Stunde bei 80°C nachgerührt und dann abgekühlt. Die kalte Suspension wurde filtriert und mit 40 Liter Wasser gewaschen, danach im Vakuum-Trockenschrank 16 Stunden bei 80°C getrocknet und wie unter A beschrieben reduziert und passiviert. Der Katalysator hatte einen Ru-Gehalt von 5,0 Gew.-%, einen Fe-Gehalt von 1,0 Gew.-% und einen Na-Gehalt von 0,036 Gew.-%.

### Beispiel 2 (Analog Beispiel 2 der EP 1 317 959)

62 g Aktivkohlestränge (Supersorbon SX 30 der Fa. Lurgi, Durchmesser 3 mm, Oberfläche ca. 1000 m²/g) wurden mit 400 ml VE-Wasser in einem Rührkessel vorgelegt und unter leichtem Rühren und Rückflusskühlung auf 80°C erhitzt. Eine Lösung aus 8,13 g Rutheniumchlorid und 3,19 g Eisenchlorid wurde bei 80°C innerhalb von 60 Minuten zugetropft. Dann wurde durch Zugabe von 1 M Natronlauge der pH-Wert auf 9 angehoben und eine Stunde nachgerührt. Der Katalysator wurde auf eine Glasfilternutsche überführt, mit 10 Liter VE-Wasser gewaschen und anschliessend im Vakuum-Trockenschrank 6 Stunden bei 80°C getrocknet. Dann wurde in einem Reduktionsofen für 3 Stunden bei 200°C in einem Gasgemisch aus Wasserstoff und Stickstoff (4150) reduziert, auf Raumtemperatur abgekühlt und mit einem Gasgemisch aus 1% Sauerstoff in Stickstoff passiviert.

### Beispiel 3 (Analog Beispiel 1D der EP 1 317 959)

50 g Noritkohle SX plus wurden mit 300 ml dest. Wasser aufgerührt und auf 80°C aufgeheizt. Innerhalb von ca. 70 Minuten wurden bei einem pH-Wert von 9,0 (gehalten über Zugabe von NaOH, 1,5 molar) Eisennitrat und Ruthenium-nitrosylnitrat in Wasser zudosiert. Nach 1 Stunde Nachrühren wurde die Kohle abfiltriert und mit ca. 16 I Wasser nachgewaschen. Danach wurde der Katalysator bei 100°C für 10 Stunden im Vakuumtrockenschrank getrocknet. Die Reduktion erfolgte im Drehrohrofen, in den zunächst der Katalysator eingebracht wurde, und dann wurde der Ofen im N₂-Strom auf 180°C aufgeheizt, 2 Stunden wurden 180 im N₂-Strom gehalten (35 Nl/h), danach wurde mit 30 Nl/h N₂ und 4 NI/h H₂ auf 200°C aufgeheizt und für 2 Stunden bei dieser Temperatur gehalten und abgekühlt ebenfalls mit 30/4 NI/h N₂/H₂. Die Passivierung erfolgte im selben Apparat bei Raumtemperatur. Erst wurde ein reiner N₂-Strom (30 Nl/h) übergeleitet, der unter Beachtung der Temperatur langsam reduziert wurde auf 0, parallel dazu wurde langsam Luft zugeführt, bis am Ende bei Raumtemperatur 10 NI/h Luft für 60 Minuten zudosiert wurden. Danach wurde der Katalysator in Wasser ausgebaut.

### Beispiel 4

Es wurde wie in Beispiel 3 vorgegangen, allerdings wurde nach der Reduktion bei 200°C nicht passiviert, sondern direkt in Wasser eingefüllt.

Der resultierende Katalysator zeigte gleich gute Eigenschaften, wie der gemäß Beispiel 3.

### Vergleichsbeispiel 1 (Katalysator gemäß Beispiel 2 der EP 1 317 959)

Es wurde ein Katalysator wie unter Beispiel 2 hergestellt, jedoch erfolgte die Reduktion bei 500°C.

### Vergleichsbeispiel 2 (Katalysator gemäß Beispiel 1D der EP 1 317 959)

Es wurde wie in Beispiel 3 verfahren, nur dass die Reduktion gemäß Stand der Technik bei 500°C erfolgt, d.h. es wurde im Stickstoffstrom auf 180°C aufgeheizt, 2 Stunden gehalten (35 NI/h), danach mit 30 Nl/h N₂ und 4 Nllh H₂ auf 500°C aufgeheizt und für 2 Stunden gehalten und abgekühlt (ebenfalls mit 30/4 NI/h N₂/H₂).

### Testung

Zum Vergleich der Katalysatoren wurde jeweils die Umsetzung von Citral mit einem Katalysator gemäß Beispiel 3 bzw. Vergleichsbeispiel 2 entsprechend folgender Vorschrift durchgeführt:
In einem druckfesten Autoklaven (300 ml Volumen) wurden ca. 3 g wasserfeuchter erfindungsgemäßer Katalysator (Figur 1 - Reduzierung bei 200°C) bzw. gemäß Stand der Technik (Figur 2 - Reduzierung bei 500°C) vorgelegt (entspricht ca. 1,5 g trockenem Katalysator). Dazu wurden jeweils 105 ml Citral-N und eine Mischung aus 37,4 ml Methanol und 7,5 ml Trimethylamin gegeben. Der Autoklave wurde geschlossen, inertisiert und mit 30 bar H₂ beaufschlagt und bei drehendem Rührer auf 80°C aufgeheizt. In der ersten Stunde wurde alle 15 Minuten eine Probe über eine Fritte gezogen, danach jede Stunde. Nach ca. 6 Stunden wurde der Versuch beendet, entspannt, abgekühlt und vor dem Öffnen mit Stickstoff gespült. Die Proben wurden im Gaschromatographen analysiert.

### Ergebnis:

Der Katalysator, der gemäß vorliegender Erfindung bei 200°C reduziert wurde, ist aktiv genug, um eine vollständige Umsetzung sogar im vierten Zyklus nach 360 Minuten zu erzielen. Im ersten Zyklus wurde vollständige Umsetzung bereits nach 100 Minuten erreicht.

Im Vergleich dazu konnte mit dem Katalysator des Vergleichsbeispiels (also gemäß EP 1 317 959) im vierten Zyklus nach 360 Minuten keine vollständige Umsetzung erreicht werden. Auch im ersten Zyklus wurde vollständige Umsetzung erst nach 120 Minuten erreicht werden.

Die Bildung von Nebenprodukten ist bei beiden Katalysatoren in etwa gleich. Die Selektivität stieg während der Versuche an: im vierten Zyklus wird weder Citronellol noch Citronellal gebildet.

Zusammenfassend ist festzustellen, dass der Katalysator gemäß vorliegender Erfindung höhere Stabilität und Aktivität zeigte als der Katalysator gemäß EP 1 317 959 bei ansonsten gleich gute Eigenschaften.

## Patentansprüche

1. Verfahren zur Herstellung eines Ruthenium/Eisen/Kohlenstoffträger-Katalysators, enthaltend neben 0,1 bis 10 Gew.-% Ruthenium auf einem Kohlenstoffträger 0,1 bis 5 Gew.-% Eisen, durch
a) Einbringen eines Trägers in Wasser
b) Simultane Zugabe der katalytisch aktiven Komponenten Ruthenium und Eisen in Form von Lösungen ihrer Metallsalze
c) gleichzeitige Auffällung der katalytisch aktiven Komponenten auf den Träger durch Zugabe einer Base
d) Abtrennen des Katalysators von der wässrigen Phase der Trägersuspension
e) Trocknen des Katalysators
f) Reduktion des Katalysators im Wasserstoffstrom bei 100 bis unter 400°C,
g) Ausbau des Katalysators unter schwer-entflammbaren Flüssigkeiten oder
passivieren des Katalysators durch Überströmen mit einem verdünnten Sauerstoffstrom
oder
passivieren des Katalysators durch Überströmen mit einem verdünnten Sauerstoffstrom und Ausbau des Katalysators unter schwer-entflammbaren Flüssigkeiten.

2. Verfahren gemäß Anspruch 1 , **dadurch gekennzeichnet, dass** die Reduktion in Schritt f) bei 120 bis 300°C, besonders bevorzugt bei 150 bis 250°C, insbesondere bevorzugt bei 180 bis 220°C durchgeführt wird.

3. Verfahren gemäß Anspruch 1 oder 2, **dadurch gekennzeichnet, dass** der hergestellte Katalysator ein Suspensionskatalysator ist.

4. Verfahren gemäß einem der Ansprüche 1 bis 3, **dadurch gekennzeichnet, dass** der hergestellte Katalysator ein Festbettkatalysator ist.

5. Verfahren gemäß einem der Ansprüche 1 bis 4, **dadurch gekennzeichnet, dass** die Schritte (b) und (c) bei einer Temperatur von 50 bis 95°C durchgeführt werden.

6. Verfahren gemäß einem der Ansprüche 1 bis 5, **dadurch gekennzeichnet, dass** die Schritte (b) und (c) sowohl gleichzeitig als auch nacheinander erfolgen können.

7. Verfahren gemäß einem der Ansprüche 1 bis 6, **dadurch gekennzeichnet, dass** die katalytisch aktiven Komponenten in Form ihrer Chloride, Nitrate, Nitrosylnitrate, Acetate, Oxide, Hydroxide oder Acetylacetonate eingesetzt werden.

8. Verfahren gemäß einem der Ansprüche 1 bis 7, **dadurch gekennzeichnet, dass** der Kohlenstoffträger durch Oxidation mit HNO₃, Sauerstoff, Wasserstoffperoxid oder Salzsäure vorbehandelt werden kann.

9. Verfahren gemäß einem der Ansprüche 1 bis 8, **dadurch gekennzeichnet, dass** zum Ausfällen der katalytisch aktiven Komponenten auf den Träger als Base Na₂CO₃, NaHCO₃, (NH₄)₂CO₃, NH₃, Harnstoff, NaOH, KOH oder LiOH verwendet wird.

10. Verfahren gemäß einem der Ansprüche 1 bis 9 **dadurch gekennzeichnet, dass** zum Ausfällen der katalytisch aktiven Komponenten NaOH verwendet wird.

11. Verfahren zur selektiven Flüssigphasenhydrierung von Carbonylverbindungen der allgemeinen Formel I 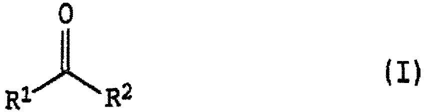 wobei
R¹, R² jeweils unabhängig voneinander gleich oder verschieden sein können und Wasserstoff oder einen gesättigten oder einen ein- oder mehrfach ungesättigten geradkettigen oder verzweigten gegebenenfalls substituierten C₁-C₂₀-Alkylrest, einen gegebenenfalls substituierten Arylrest oder eine gegebenenfalls substituierte heterocyclische Gruppe darstellen,
zu den entsprechenden Alkoholen der allgemeinen Formel II 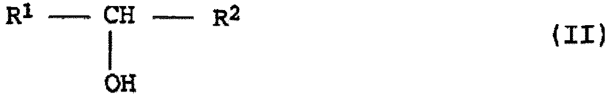
wobei R¹ und R² die oben angegebene Bedeutung haben, bei dem man
(i) einen Katalysator nach einem Verfahren gemäß einem der Ansprüche 1 bis 10 herstellt, und
(ii) die Carbonylverbindung an dem Katalysator hydriert.

12. Verfahren gemäß Anspruch 11 , **dadurch gekennzeichnet, dass** die Carbonylverbindung eine alpha,beta-ungesättigte Carbonylverbindung ist.

13. Verfahren gemäß einem der Ansprüche 11 oder 12, **dadurch gekennzeichnet, dass** die Carbonylverbindung Citral ist.

14. Verfahren gemäß Anspruch 11 , **dadurch gekennzeichnet, dass** die Carbonylverbindung Citronellal ist.

## Claims

1. A process for producing a ruthenium-iron-carbon support catalyst comprising from 0.1 to 5% by weight of iron in addition to from 0.1 to 10% by weight of ruthenium on a carbon support by
a) introduction of a support into water
b) simultaneous addition of the catalytically active components ruthenium and iron in the form of solutions of their metal salts
c) coprecipitation of the catalytically active components on the support by addition of a base
d) separation of the catalyst from the aqueous phase of the support suspension
e) drying of the catalyst
f) reduction of the catalyst in a stream of hydrogen at 100 to below 400°C
g) deinstallation of the catalyst under relatively nonflammable liquids
or
passivation of the catalyst by passing a diluted oxygen stream over it
or
passivation of the catalyst by passing a diluted oxygen stream over it and deinstallation of the catalyst under relatively nonflammable liquids.

2. The process according to claim 1, wherein the reduction in step f) is carried out at from 120 to 300°C, particularly preferably from 150 to 250°C, preferred in particular from 180 to 220°C.

3. The process according to claim 1 or 2, wherein the catalyst produced is a suspension catalyst.

4. The process according to any of claims 1 to 3, wherein the catalyst produced is a fixed-bed catalyst.

5. The process according to any of claims 1 to 4, wherein steps (b) and (c) are carried out at a temperature of from 50 to 95°C.

6. The process according to any of claims 1 to 5, wherein steps (b) and (c) can be carried out either simultaneously or successively.

7. The process according to any of claims 1 to 6, wherein the catalytically active components are used in the form of their chlorides, nitrates, nitrosyl nitrates, acetates, oxides, hydroxides or acetylacetonates.

8. The process according to any of claims 1 to 7, wherein the carbon support can be pretreated by oxidation by means of HNO₃, oxygen, hydrogen peroxide or hydrochloric acid.

9. The process according to any of claims 1 to 8, wherein Na₂CO₃, NaHCO₃, (NH₄)₂CO₃, NH₃, urea, NaOH, KOH or LiOH is used as base for precipitation of the catalytically active components onto the support.

10. The process according to any of claims 1 to 9, wherein NaOH is used for precipitation of the catalytically active components.

11. A process for the selective liquid-phase hydrogenation of carbonyl compounds of the general formula I 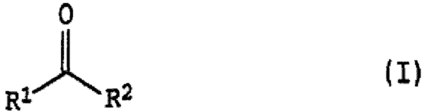 where
R¹, R² can each be, independently of one another, identical or different and are each hydrogen or a saturated or a monounsaturated or polyunsaturated straight-chain or branched, optionally substituted, C₁-C₂₀-alkyl radical, an optionally substituted aryl radical or an optionally substituted heterocyclic group, to the corresponding alcohols of the general formula II 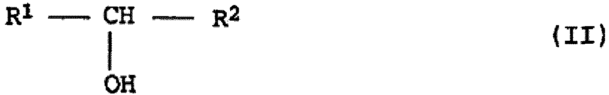
where R¹ and R² are as defined above, in which
(i) a catalyst is prepared by a process according to any of claims 1 to 10, and
(ii) the carbonyl compound is hydrogenated over the catalyst.

12. The process according to claim 11, wherein the carbonyl compound is an alpha,beta-unsaturated carbonyl compound.

13. The process according to either of claims 11 and 12, wherein the carbonyl compound is citral.

14. The process according to claim 11, wherein the carbonyl compound is citronellal.

## Revendications

1. Procédé pour la production d'un catalyseur à base de ruthénium/fer sur support en carbone, contenant, outre 0,1 à 10 % en poids de ruthénium sur un support en carbone, 0,1 à 5 % en poids de fer, par
a) introduction d'un support dans de l'eau
b) addition simultanée des composants catalytiquement actifs ruthénium et fer sous forme de solutions de leurs sels métalliques
c) précipitation simultanée des composants catalytiquement actifs sur le support par addition d'une base
d) séparation du catalyseur d'avec la phase aqueuse de la suspension de support
e) séchage du catalyseur
f) réduction du catalyseur dans le courant d'hydrogène à une température de 100 à moins de 400 °C,
g) achèvement du catalyseur sous l'effet de liquides difficilement inflammables
ou
passivation du catalyseur en faisant passer sur ce dernier un courant d'oxygène dilué
ou
passivation du catalyseur en faisant passer sur ce dernier un courant d'oxygène dilué et achèvement du catalyseur sous l'effet de liquides difficilement inflammables.

2. Procédé selon la revendication 1, **caractérisé en ce que** la réduction dans l'étape f) est effectuée à une température de 120 à 300 °C, en particulier de préférence à une température de 150 à 250 °C, de façon particulièrement préférée de 180 à 220 °C.

3. Procédé selon la revendication 1 ou 2, **caractérisé en ce que** le catalyseur produit est un catalyseur en suspension.

4. Procédé selon l'une quelconque des revendication 1 à 3, **caractérisé en ce que** le catalyseur produit est un catalyseur en lit fixe.

5. Procédé selon l'une quelconque des revendication 1 à 4, **caractérisé en ce que** les étapes (b) et (c) sont effectuées à une température de 50 à 95 °C.

6. Procédé selon l'une quelconque des revendication 1 à 5, **caractérisé en ce que** les étapes (b) et (c) peuvent s'effectuer aussi bien simultanément que l'une après l'autre.

7. Procédé selon l'une quelconque des revendication 1 à 6, **caractérisé en ce que** les composants catalytiquement actifs sont utilisés sous forme de leurs chlorures, nitrates, nitrates de nitrosyle, acétates, oxydes, hydroxydes ou acétylacétonates.

8. Procédé selon l'une quelconque des revendication 1 à 7, **caractérisé en ce que** le support en carbone peut être prétraité par oxydation par HNO₃, l'oxygène, le peroxyde d'hydrogène ou l'acide chlorhydrique.

9. Procédé selon l'une quelconque des revendication 1 à 8, **caractérisé en ce que** pour la précipitation des composants catalytiquement actifs sur le support on utilise comme base Na₂CO₃, NaHCO₃, (NH₄)₂CO₃, NH₃, l'urée, NaOH, KOH ou LiOH.

10. Procédé selon l'une quelconque des revendication 1 à 9, **caractérisé en ce que** pour la précipitation des composants catalytiquement actifs on utilise NaOH.

11. Procédé pour l'hydrogénation sélective en phase liquide de composés carbonyle de formule générale I 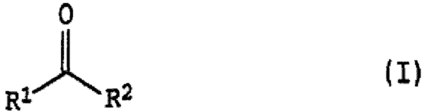 dans laquelle
R¹, R² indépendamment l'un de l'autre, peuvent être identiques ou différents et représentent chacun un atome d'hydrogène ou un radical alkyle en C₁-C₂₀ saturé ou une ou plusieurs fois insaturé, à chaîne droite ou ramifié, éventuellement substitué, un radical aryle éventuellement substitué ou un groupe hétérocyclique éventuellement substitué,
conduisant aux alcools correspondants de formule générale II 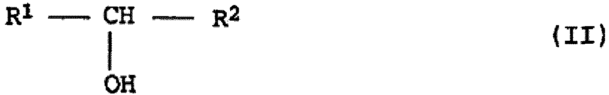
dans laquelle R¹ et R² ont la signification indiquée plus haut, dans lequel on
(i) produit un catalyseur conformément à un procédé selon l'une quelconque des revendications 1 à 10, et
(ii) soumet le composé carbonyle à une hydrogénation sur le catalyseur.

12. Procédé selon la revendication 11, **caractérisé en ce que** le composé carbonyle est un composé carbonyle alpha,bêta-insaturé.

13. Procédé selon la revendication 11 ou 12, **caractérisé en ce que** le composé carbonyle est le citral.

14. Procédé selon la revendication 11, **caractérisé en ce que** le composé carbonyle est le citronellal.
